# EUROPEAN PATENT APPLICATION

(11) **EP 3 747 458 A1**
(43) Date of publication of application: **09.12.2020**
(21) Application number: 19746764.0
(22) Date of filing: 09.01.2019
(51) Int. Cl.: A61K 38/20, A61P 35/00, A61P 35/04

(54) **ANTITUMOR PROTEIN COMPOSITION AND APPLICATION THEREOF**

(30) Priority: 01.02.2018 CN 201810104146
(71) Applicant: Beijing Kenuokefu Biotechnology Co., Ltd., Beijing 102200 (CN)
(72) Inventor: ZHANG, Jinyu, Beijing 102200 (CN)
(74) Representative: Dr. Gassner & Partner mbB
(86) International application number: PCT/CN2019/070994
(87) International publication number: WO 2019/149039

(57) **Abstract**

An antitumor pharmaceutical composition, comprising proteins IL 12, GMCSF, and IL 2. The present application has technical effects of effectively inhibiting tumors at various stages with mild adverse reactions.

## Description

### TECHNICAL FIELD

This application relates to a field of an anti-tumor drug, and more particularly to an anti-tumor protein composition and application thereof.

### BACKGROUND

Tumor is a neogrowth formed by clonal hyperplasia when a cell of local tissue cannot regulate its normal growth at the genetic level due to various carcinogenic factors, and tumor is also called neoplasm since most of the neogrowths are protruded as a space-occupying lump.

In resent years, there are many new ways developed for tumor therapy. Radiotherapy, chemotherapy, surgical therapy, and immunotherapy are current commonly-used therapeutic means. However, problems such as serious adverse reactions during radiotherapy and chemotherapy, high risk caused by surgical therapy, and poor effect of the immunotherapy against solid tumors still exist.

### BRIEF SUMMARY

A first object of the present application is to provide an anti-tumor protein composition having the following advantages: 1. minor adverse reactions; 2. good inhibitory effect against various solid tumors which can reduce or even eliminate the tumor; 3. good inhibitory effect against the metastasis of malignant tumors; and 4. easy implementation by existing technologies.

The above object of the present invention can be achieved by the technical solutions as follow: an anti-tumor drug composition including proteins IL12, GMCSF, and IL2.

By the above-mentioned technical solution, the symptoms in patients suffering from a plurality of solid tumors can be well controlled, and some conditions can even be completely relieved. It has little stimulation to a patient and minor adverse reactions, which greatly improves the life quality of the patient.

Preferably, in the composition, a mass ratio of the IL12 protein: the GMCSF protein: the IL2 protein is in ranges of 0.1-10:0.1-10:0.1-10.

Preferably, in the composition, the mass ratio of the IL12 protein: the GMCSF protein : the IL2 protein is in ranges of 0.6-6:0.6-6:0.6-6.

Preferably, in the composition, the mass ratio of the IL12 protein: the GMCSF protein : the IL2 protein is 1:6:2.

A second object of this application is to provide a polynucleotide composition including one of the following:
(a) a polynucleotide composition coding proteins IL12, GMCSF, and IL2 in claim 1; and
(b) a polynucleotide composition complementary to the polynucleotide (a).

A third object of this application is to provide a recombinant vector composition, which is constructed from each polynucleotide of the polynucleotide composition in claim 5 with a plasmid, a virus, and an expressing vector, respectively.

A fourth object of this application is to provide a genetically engineered host cell composition, wherein a host cell of the host cell composition includes one of the following: (a) a host cell transformed or transduced from the recombinant vector in claim 6; and (b) a host cell transformed or transduced from the polynucleotide composition in claim 5.

A fifth objection of this application is to provide an anti-tumor drug including the anti-tumor drug composition in any of claims 1-4, or the polynucleotide composition in claim 5, or the recombinant vector composition in claim 6, or the host cell composition in claim 7, and a pharmaceutically acceptable carrier or buffer or additive or excipient or adjuvant.

A six object of this application is to provide an application of the anti-tumor drug composition in any of claims 1-4, or the polynucleotide composition in claim 5, or the recombinant vector composition in claim 6, or the host cell composition in claim 7, or the anti-tumor drug in claim 8 in a field of a drug treating tumors in a mammal.

Preferably, the mammal includes Diprotodontia other than human beings, Carnivora, Perissodactyla, Artiodactyla, Rodentia, and Lagomorpha.

Preferably, the tumor includes melanoma, kidney cancer, prostate cancer, breast cancer, colon cancer, lung cancer, liver cancer, bone cancer, pancreatic cancer, skin cancer, head or neck cancer, oral cancer, nasopharyngeal cancer, skin or intraocular malignant melanoma, uterine cancer, ovarian cancer, rectal cancer, anal cancer, gastric cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, thyroid cancer, parathyroid cancer, adrenal cancer, soft tissue sarcoma, urethral cancer, penile cancer, acute myeloid leukemia, chronic myeloid leukemia, acute lymphoblastic leukemia, chronic lymphocytes leukemia, lymphocytic lymphoma, bladder cancer, renal or ureteral cancer, renal pelvis cancer, central nervous system neoplasm, primary central nervous system lymphoma, tumor angiogenesis, spinal cord tumor, brain stem glioma, pituitary adenoma, Kaposi's sarcoma, epidermoid carcinoma, squamous cell carcinoma, and T cell lymphoma.

A seventh object of this application is to provide a anti-tumor drug composition in any of claims 1-4, or the polynucleotide composition in claim 5, or the recombinant vector composition in claim 6, or the host cell composition in claim 7, or the anti-tumor drug in claim 8, which is injected into an affected area of animal or introduced into an affected area of a tumor by intravenous injection through a targeted carrier.

In summary, the embodiments of the present application have the following beneficial effects:
1. minor adverse reactions, which is commonly a temporal fever;
2. good inhibitory effect against various solid tumors which can reduce or even eliminate the tumor;
3. good inhibitory effect against the metastasis of malignant tumors; and
4. greatly improved life quality of a patient.

### BRIEF DESCRIPTION OF DRAWINGS

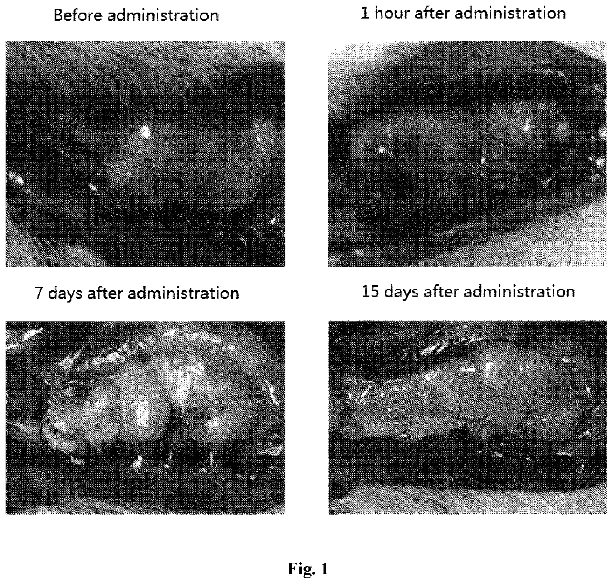

Fig. 1 is a diagram showing the treatment effect on an experimental animal in Example 5;

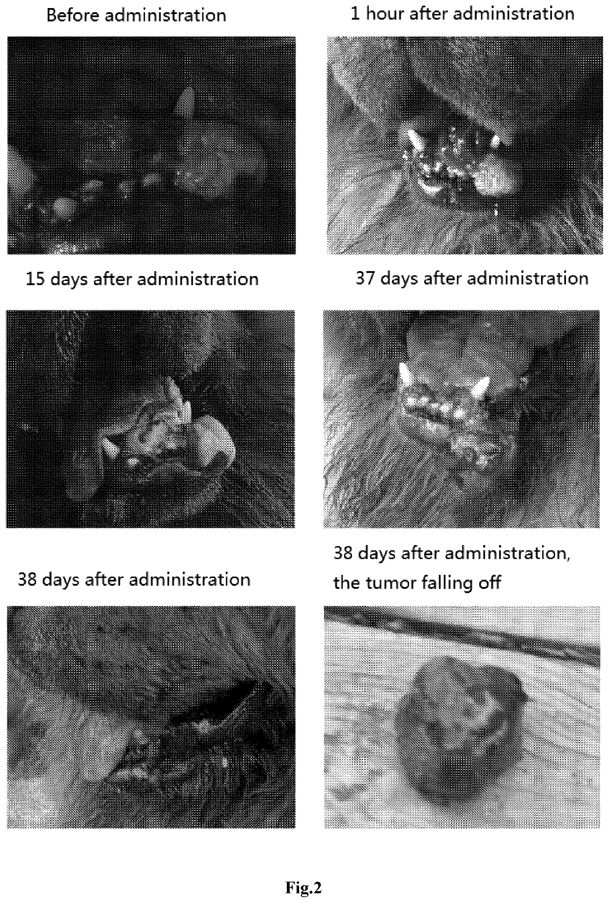

Fig. 2 is a diagram showing the treatment effect on an experimental animal in Example 6;

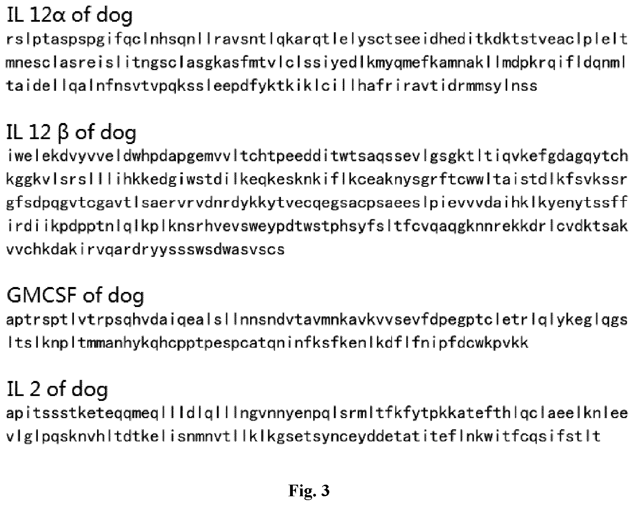

Fig. 3 shows sequences of dog proteins IL12, GMCSF, and IL2; and

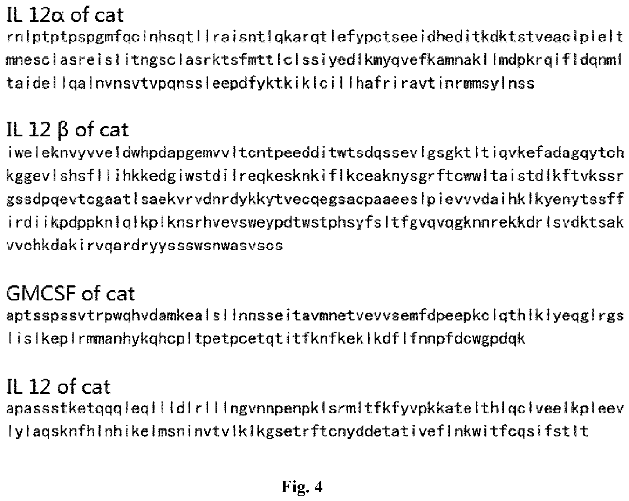

Fig. 4 shows sequences of cat proteins IL12, GMCSF, and IL2, which are consistent with those provided in the nucleotide sequence list attached hereto.

### DETAILED DESCRIPTION

This application will be further explained in detail as follow.

Agents: DMEM medium, 1640 medium, fetal bovine serum purchased from lifetechnologies company; CDM4HEK293 serum free medium purchased from Thermo company; cell culture flask and culture plate purchased from Corning company; Puromycin purchased from Chemicon company; restriction endonuclease purchased from Takara and NEB company; ligase purchased from NEB company; DNA polymerase purchased from Takara company; Plasmid Extraction Kit and Gel Extraction Kit purchased from OmegaBiotech company; primer synthesized by Sangon Biotech (Shanghai) Co., Ltd; and gene synthesis and sequencing approached by lifetechnologies company. IL12 ELISA kit and IL2 ELISA kit purchased from Thermo company; GMCSF ELISA kit purchased from Sigma company; and chitosan (Protosan G 213) purchased from NovaMatrix company. Recombinant dogs IL12, GMCSF, IL2 protein, recombinant cats IL12, GMCSF, IL2 protein, purchased from NovusBiologicals company.

### Example 1: Construction of cells expressing IL12

The coding region of dog IL12 gene including two subunits IL12α(Genbank No: NM_001003293) and IL12β(Genbank No: NM_001003292) was synthesized and these two subunits were ligated by T2A sequence. Two ends of the synthesized gene include restriction sites BamHI and XhoI, respectively. Then the synthesized gene was cleaved by BamHI and XhoI via the following system: 5µg IL12 plasmid, 4µL digestion buffer, 1µL BamHI, 1µL XhoI, a total volume of 40µL obtained by adding water, and 12 hours of standing at 37°C. The Eppendorf was taken out, and 4.4 µL 10^{∗}loading buffer was added to the eppendorf. An electrophoresis was carried out by using 1% agarose gel, after which fragments of IL12 gene were recovered for use.

Expression vector pLentis-CMV-MCS-IRES-PURO was cleaved via the following system: 2µg plasmid, 3µL digestion buffer, 1µL BamHI, 1µL XhoI, a total volume of 30µL obtained by adding water, and 12 hours of standing at 37°C. Eppendorf was taken out, and 3.3 µL 10^{∗}loading buffer was added to the eppendorf. An electrophoresis was carried out by using 1% agarose gel, after which fragments of the vector were recovered for use.

PLentis-CMV-MCS-IRES-PURO and IL12 were ligated via the following system: 2µL pLentis-CMV-MCS-IRES-PURO, 2µL IL12, 1µL ligase buffer, 0.5µL T4 DNA ligase, and 4.5µL water. The ligation was performed at room temperature for 4 hours. Then competent cells from *Escherichia coli* were transformed into the ligated system. The next day, bacterial colonies were picked up from the transformed plate, placed in LB medium and cultured in a shaker at a temperature of 37°C overnight. Plasmid was extracted from the cultured bacterium by using the Plasmid Extraction Kit, and fragments were cleaved and identified whether they had been successfully ligated into vectors. Desired vectors were sequenced for examination, and it was confirmed that the expression vector pLentis-CMV-IL12-PGK-PURO had been successfully constructed.

Virus for regulating the vector was prepared by the following method: 1. Cultured 293FT cells were digested, counted, and placed into wells of a 10-cm culture plate by 3^{∗}10⁶ cells/well, with the volume of the culture solution being 10ml. 2. At the next night, the state of cells was observed, and desired cells were transfected. Chloroquine was added to the plate until a final concentration of 25µm was reached. Sterile water and a plasmid (pMD2.G 5µg+pSPAX2 15 µg+pLentis-CMV-IL12-PGK-PURO 20µg) were added to a test tube to reach a total volume of 1045µL. Then 155µL of 2M CaCl₂ was added to the test tube and mixed evenly. Finally 1200µL 2^{∗}HBS was added to the test tube under shaking. The mixture was quickly added into the wells of the plate, and mixed evenly under a gentle shaking. 3. On the morning of the third day, the state of cells was observed, and the medium was replaced by 10ml fresh DMEM medium. 4. On the morning of the fifth day, the state of cells was observed, and supernatant in the plate was collected, filtered through a 0.45µm filter, and centrifuged for 2 hours in a high-speed centrifuge tube at 50000g. The supernatant was carefully removed. The precipitate was dried by an absorbent paper, resuspended in 500µL HBSS for 2 hours, sub-packed in several tubes and preserved at -70°C.

Virus was used to transfect 293 cells by the following method: cultured 293 cells were digested, and inoculated into the wells of a 6-well plate by 10⁵ cells/well, in which the volume of the culture solution was 1ml. After 24 hours, 10µL virus for regulating the vector was added, and cells were cultured in the incubator for another 24 hours. Then, the supernatant was removed, and fresh medium was added. After the surface was fully covered with cells, cells were transferred to a flask. Puromycin at a suitable concentration was added to the flask, and culturing was continued, during which the medium was replaced every two days and the concentration of puromycin was kept at 3µg/ml. After one week of screening, viable cells were acquired, that is, cells stably expressing regulation protein, which were named 293(IL12).

### Example 2: construction of cells expressing GMCSF

The coding region of dog GMCSF gene (Genbank No: NM_001003245) was synthesized. Two ends of the synthesized gene include restriction sites BamHI and XhoI, respectively. Then the synthesized gene was cleaved by BamHI and XhoI via the following system: 5µg GMCSF plasmid, 4µL digestion buffer, 1µL BamHI, 1µL XhoI, a total volume of 40µL obtained by adding water, and 12 hours of standing at 37°C. Eppendorf was taken out, and 4.4 µL 10^{∗}loading buffer was added to the eppendorf. An electrophoresis was carried out by 1% using agarose gel, after which the fragments of GMCSF gene were recovered for use.

Expression vector pLentis-CMV-MCS-IRES-PURO was cleaved via the following system: 2µg plasmid, 3µL digestion buffer, 1µL BamHI, 1µL XhoI, a total volume of 30µL obtained by adding water, and 12 hours of standing at 37°C. Eppendorf was taken out, and 3.3 µL 10^{∗}loading buffer was added to the eppendorf. An electrophoresis was carried out by using 1% agarose gel, and fragments of the vector were recovered for use.

PLentis-CMV-MCS-IRES-PURO and GMCSF were ligated via the following system: 2µL pLentis-CMV-MCS-IRES-PURO, 2µL GMCSF, 1µL ligase buffer, 0.5µL T4 DNA ligase, and 4.5µL water. The ligation was performed at room temperature for 4 hours. Then competent cells from Escherichia coli were transformed into the ligated system. The next day, bacterial colonies were picked up from the transformed plate, placed in LB medium and cultured in a shaker at a temperature of 37°C overnight. Plasmid was extracted from the cultured bacterium by using the Plasmid Extraction Kit, and fragments were cleaved and identified whether they had been successfully ligated into the vectors. Desired vectors were sequenced for examination, and it was confirmed that the expression vector pLentis-CMV-GMCSF-PGK-PURO had been successfully constructed.

Virus for regulating the vector was prepared by the following method: 1. cultured 293FT cells were digested, counted, placed into the wells of a 10-cm culture plate by 3^{∗}10⁶ cells/well, in which the volume of the culture solution was 10ml. 2. At the next night, the state of cells was observed, and desired cells were transfected. Chloroquine was added to the plate until a final concentration of 25µm was reached. Sterile water and a plasmid (pMD2.G 5µg+pSPAX2 15 µg+pLentis-CMV-IL12-PGK-PURO 20µg) were added to a test tube to reach a total volume of 1045µL. Then 155µL of 2M CaCl₂ was added to the test tube and mixed evenly. Finally 1200µL 2^{∗}HBS was added to the test tube under shaking. The mixture was quickly added into the wells of the plate, and mixed evenly under a gentle shaking. 3. On the morning of the third day, the state of cells was observed, and the medium was replaced by 10ml fresh DMEM medium. 4. On the morning of the fifth day, the state of cells was observed, and supernatant in the plate was collected, filtered through 0.45µm filter, and centrifuged in a high-speed centrifuge tube at 50000g for 2 hours. The supernatant was carefully removed, and the precipitate was dried by an absorbent paper, resuspended in 500µL HBSS for 2 hours, sub-packed into several tubes and preserved at -70°C.

Virus was used to transfect 293 cells by the following method: cultured 293 cells were digested, and inoculated into a 6-well plate by 10⁵ cells/well, in which the volume of the culture solution was 1ml. After 24 hours, 10µL virus for regulating the vector was added, and cells were cultured in the incubator for another 24 hours. Then, the supernatant was removed, and fresh medium was added. After the surface was fully covered with cells, cells were transferred into a flask. Puromycin at a suitable concentration was added, and culturing was continued, during which medium was replaced every two days, and the concentration of puromycin was kept at 3µg/ml. After one week of screening, viable cells were acquired, that is, cells stably expressing regulation protein, which were named 293(GMCSF).

### Example 3: construction of cells expressing IL2

The coding region of dog IL2 gene (Genbank No: NM_001003305) was synthesized. Two ends of the synthesized gene include restriction sites BamHI and XhoI, respectively. Then the synthesized gene was cleaved by BamHI and XhoI via the following system: 5µg IL2 plasmid, 4µL digestion buffer, 1µL BamHI, 1µL XhoI, a total volume of 40µL obtained by adding water, and 12 hours of standing at 37°C. Eppendorf was taken out, and 4.4 µL 10^{∗}loading buffer was added to the eppendorf. An electrophoresis was carried out by using 1% agarose gel, and fragments of IL2 gene were recovered for use.

Expression vector pLentis-CMV-MCS-IRES-PURO was cleaved via the following system: 2µg plasmid, 3µL digestion buffer, 1µL BamHI, 1µL XhoI, a total volume of 30µL obtained by adding water, and 12 hours of standing at 37°C. Eppendorf was taken out, and 3.3 µL 10^{∗}loading buffer was added to the eppendorf. An electrophoresis was carried out by using 1% agarose gel, and the fragments of vector were recovered for use.

PLentis-CMV-MCS-IRES-PURO and IL2 were ligated via the following system: 2µL pLentis-CMV-MCS-IRES-PURO, 2µL IL2, 1µL ligase buffer, 0.5µL T4 DNA ligase, and 4.5µL water. The ligation was performed at room temperature for 4 hours. Then competent cells from Escherichia coli were transformed into the ligated system. The next day, bacterial colonies were picked up from the transformed plate, placed in LB medium and cultured in a shaker at a temperature of 37°C overnight. Plasmid was extracted from the cultured bacterium by using the Plasmid Extraction Kit, and fragments were cleaved and identified whether they had been successfully ligated into vectors. Desired vectors were sequenced for examination, and it was confirmed that the expression vector pLentis-CMV-IL2-PGK-PURO had been successfully constructed.

Virus for regulating the vector was prepared by the following method: 1. cultured 293FT cells were digested, counted, and placed into a 10-cm culture plate by 3^{∗}10⁶ cells/well, in which the volume of the culture solution was 10ml. 2. At the next night, the state of cells was observed, and desired cells were transfected. Chloroquine was added to the plate until a final concentration of 25µm was reached. Sterile water and a plasmid (pMD2.G 5µg+pSPAX2 15 µg+pLentis-CMV-IL12-PGK-PURO 20µg) were added to a test tube to reach a total volume of 1045µL. Then 155µL of 2M CaCl₂ was added to the test tube and mixed evenly. Finally 1200µL 2^{∗}HBS was added under shaking. The mixture was quickly added into the wells of the plate, and mixed evenly under a gentle shaking. 3. On the morning of the third day, the state of cells was observed, and the medium was replaced by 10ml fresh DMEM medium. 4. On the morning of the fifth day, the state of cells was observed, and supernatant in the plate was collected, filtered through 0.45µm filter, and centrifuged in a high-speed centrifuge tube at 50000g for 2 hours. The supernatant was carefully removed, and the precipitate was dried by an absorbent paper, resuspended in 500µL HBSS for 2 hours, sub-packed into several tubes and preserved at -70°C.

Virus was used to transfect 293 cells by the following method: cultured 293 cells were digested, and inoculated into a 6-well plate by 10⁵ cells/well, in which the volume of the culture solution was 1ml. After 24 hours, 10µL virus for regulating the vector was added, and the culturing was continued in the incubator for another 24 hours. Then, the supernatant was removed, and fresh medium was added. After the surface was fully covered with cells, cells were transferred into a flask. Puromycin at a suitable concentration was added, and culturing was continued, during which the medium was replaced every two days, and the concentration of puromycin was kept at 3µg/ml. After one week of screening, viable cells were acquired, that is, cells stably expressing regulation protein, which were named 293 (IL2).

### Example 4: preparation of protein solution

Cultured cells 293(IL12), 293(GMCSF), and 293(IL2) were transferred to a 15cm culturing dish, respectively, in which the medium was complete medium having volume of 25ml. When the density of cells reached 90% or above, the complete medium was replaced by 25ml CDM4HEK293 serum free medium and culturing was continued for another 96 hours. The supernatant was collected, centrifuged at 1000rpm for 10min, and filtered through 0.22µm filter. Filtered solution was concentrated by an Amicon µLtra-15 ultrafiltration tube to one twentieth of the original volume. The concentration of target proteins in the concentrated solution was detected by ELISA kit. The concentration of IL12 was 100ng/µL, the concentration of GMCSF was 600ng/µL, and the concentration of IL2 was 200ng/µL.

### Example 5: Therapy 1 of tumor in dogs

Male hybrid dog, 9 years old, 2 sarcomas outside the gingiva on the left upper jaw, the size of the anterior tumor was 25mm^{∗}15mm, and the size of the posterior tumor was 30mm^{∗}20mm. As shown in Table 1, after the first administration, the anterior tumor was eliminated, and the area of the posterior tumor was reduced by 70%. Transient fever appeared 8 hours after the administration, body temperature increased by 1°C and returned to normal after 3 hours, and then all signs became normal.

A 3% sterile chitosan solution was prepared in advance for use. The dosage of drugs to be injected was prepared according to the area of the tumor, and the total dosage was 1µL/1mm² tumor. For a tumor with a size of about 900mm², the injection dosage was 900µL, including 1/6 volume of IL12 solution, 1/6 volume of GMCSF solution, 1/6 volume of IL2 solution, and 1/2 volume of 3% chitosan solution. 150 µL IL12 solution, 150 µL GMCSF solution, and 150 µL IL2 solution were mixed evenly, then 450µL 3% chitosan solution was added, and mixed evenly under slow blowing to avoid bubbles. After the dog was anesthetized, prepared drug solution was injected into the tumor slowly, and the physiological state of the dog was monitored.

**Table 1: table of size of tumor after the drug was injected in example 5**

| Time (day) | 0 | 7 | 14 | 21 | 28 | 35 |
|---|---|---|---|---|---|---|
| Anterior tumor (mm) | 25^{∗}15 | 10^{∗}6 | 0 | 0 | 0 | 0 |
| Posterior tumor (mm) | 30^{∗}20 | 25^{∗}15 | 20^{∗}15 | 15^{∗}10 | 15^{∗}10 | 15^{∗}10 |

### Example 6: Therapy 2 of tumor in dogs

Canine, chow chow, 11 years old, male, oral melanoma, 2 lesions, 40 ^{∗} 30mm inside the mandibular gingiva, and 30 ^{∗} 25mm outside the mandibular gingiva. As shown in Table 2, after one dose of the drug was injected to melanomas inside and outside the gingiva, respectively, the melanoma inside the gingiva disappeared, and the melanoma outside the gingiva fell off from the root. There was no fever or any adverse reactions observed after administration.

The IL12 solution and IL2 solution were further ultrafiltered and concentrated to 300 ng/µL, and the GMCSF solution was diluted to 300 ng/µL. Then the drug was injected by IL12: GMCSF: IL2: 3% chitosan = 1: 1: 1: 3, and the total injection volume was 1 µL/mm² tumor. For a tumor with a size of about 900mm², the injection dosage was 900µL, including 45µg IL12, 45µg GMCSF, and 45µg IL2. After the dog was anesthetized, prepared drug solution was injected into the tumor slowly, and the physiological state of the dog was monitored.

**Table 2: table of size of the tumor after the drug was injected in Example 6**

| Time (day) | 0 | 7 | 14 | 21 | 28 | 35 | 42 |
|---|---|---|---|---|---|---|---|
| Inside tumor (mm) | 40^{∗}30 | 40^{∗}30 | 30^{∗}25 | 21^{∗}16 | 12^{∗}8 | 0 | 0 |
| Outside tumor (mm) | 30^{∗}25 | 30^{∗}25 | 30^{∗}25 | 30^{∗}25 | 30^{∗}25 | 38^{∗}31 | 0 |

### Example 7: Therapy 3 of tumor in dogs

Female hybrid dog, 15 years old, breast cancer in the third breast region on the left side, with a tumor size of 55mm ^{∗} 43mm. As shown in Table 3, the area of the tumor was reduced by 60% after one intratumoral injection. Fever appeared 8 hours after administration, body temperature increased by up to 2°C, and the body temperature returned to normal after 18 hours. Then all signs became normal with no adverse reactions.

The purchased recombinant dog IL12 was diluted with sterile deionized water to 60 ng/µL, the purchased recombinant dog GMCSF was diluted with sterile deionized water to 600ng/µL, and the purchased recombinant dog IL2 was diluted with sterile deionized water to 600 ng/µL. Then the drug was injected by IL12: GMCSF: IL2: 3% chitosan = 1: 1: 1: 3, and the total injection volume was 1 µL/mm² tumor. For a tumor with a size of about 900mm², the injection dosage was 900µL, including 9µg IL12, 90µg GMCSF, and 90µg IL2. After the dog was anesthetized, prepared drug solution was injected into the tumor slowly, and the physiological state of the dog was monitored. Changes in the size of the tumor were recorded, and the efficacy was evaluated.

**Table 3: table of size of tumor after being injected in Example 7**

| Time(day) | 0 | 7 | 14 | 21 | 28 | 35 |
|---|---|---|---|---|---|---|
| Size of tumor (mm) | 55^{∗}43 | 61^{∗}49 | 50^{∗}42 | 45^{∗}38 | 39^{∗}33 | 33^{∗}28 |

### Example 8: Therapy 4 of tumor in dogs

Female hybrid dog, 10 years old, stromal sarcoma on left front leg, 35mm^{∗}35mm^{∗}40mm. As shown in Table 4, after one intratumoral injection, the area of the tumor was reduced by 60%, and the volume of the tumor was reduced by 50%. Fever appeared 10 hours after administration, body temperature increased by up to 1.5°C, and body temperature returned to normal after 8 hours. There was no adverse reactions.

The purchased recombinant dog IL12 was diluted with sterile deionized water to 300 ng/µL, the purchased recombinant dog GMCSF was diluted with sterile deionized water to 60ng/µL, and the purchased recombinant dog IL2 was diluted with sterile deionized water to 6000 ng/µL. Then the drug was injected by IL12: GMCSF: IL2: 3% chitosan = 1: 1: 1: 3, and the total injection volume was 1 µL/mm² tumor. For a tumor with a size of about 900mm², the injection dosage was 900µL, including 45µg IL12, 9µg GMCSF, and 900µg IL2. After the dog was anesthetized, prepared drug solution was injected into the tumor slowly, and the physiological state of the dog was monitored. Changes in the size of the tumor were recorded, and the efficacy was evaluated.

**Table 4: table of size of tumor after the drug injected in Example 8**

| Time (day) | 0 | 7 | 14 | 21 | 28 | 35 |
|---|---|---|---|---|---|---|
| Size of tumor(mm) | 35^{∗}35^{∗}40 | 35^{∗}35^{∗}38 | 35^{∗}35^{∗}35 | 35^{∗}35^{∗}33 | 30^{∗}30^{∗}25 | 30^{∗}30^{∗}25 |

### Example 9: Therapy 1 of tumor in cat

Female Chinese Dragen-Li, 9 years old, breast cancer, long diameter of 21mm. As shown in Table 5, after one intratumoral injection, the tumor was eliminated. There was no fever or any adverse reactions after administration.

Purchased recombinant cat IL12, GMCSF, and IL2 were diluted with sterile deionized water to 200 ng/µL. Then the drug was injected by IL12: GMCSF: IL2: 3% chitosan = 1: 1: 1: 3, and the total injection volume was 1 µL/mm² tumor. For a tumor with a size of about 900mm², the injection dosage was 900µL, including 30µg IL12, 30µg GMCSF, and 30µg IL2. After the cat was anesthetized, prepared drug solution was injected into the tumor slowly, and the physiological state of the cat was monitored. Changes in the size of the tumor were recorded, and the efficacy was evaluated.

**Table 5: table of size of tumor after the drug was injected in Example 9**

| Time(day) | 0 | 5 | 10 | 15 | 20 | 25 |
|---|---|---|---|---|---|---|
| Size of tumor(mm) | 21 | 21 | 15 | 10 | 6 | 0 |

According to the observation of animal experiments, congestion occurred in the affected area of the animal tumor after injection, then suppuration occurred, and the tumor tissue eventually fell off the animal or was eliminated. This was because that this drug composition stimulated the recognition and killing activity of the immune system of the diseased animal to tumor cells, such that the malignant tumor was inhibited, and then the tumor was reduced or even eliminated.

These embodiments are only an explanation of this application, and do not limit the protection scope of this application. Those skilled in the art can make modifications without creative contribution to this embodiment after reading this specification, and it is protected by the patent law as long as it is within the scope of the claims of this application.

序列表
<110>
<120>
<160> 8
<170> SIPOSequenceListing 1.0
<210> 1
<211> 197
<212> PRT
<213> 犬(Canis familiaris)
<400> 1
Arg Ser Leu Pro Thr Ala Ser Pro Ser Pro Gly Ile Phe Gln Cys Leu
1               5                   10                  15
Asn His Ser Gln Asn Leu Leu Arg Ala Val Ser Asn Thr Leu Gln Lys
            20                  25                  30
Ala Arg Gln Thr Leu Glu Leu Tyr Ser Cys Thr Ser Glu Glu Ile Asp
        35                  40                  45
His Glu Asp Ile Thr Lys Asp Lys Thr Ser Thr Val Glu Ala Cys Leu
    50                  55                  60
Pro Leu Glu Leu Thr Met Asn Glu Ser Cys Leu Ala Ser Arg Glu Ile
65                  70                  75                  80
Ser Leu Ile Thr Asn Gly Ser Cys Leu Ala Ser Gly Lys Ala Ser Phe
                85                  90                  95
Met Thr Val Leu Cys Leu Ser Ser Ile Tyr Glu Asp Leu Lys Met Tyr
            100                 105                 110
Gln Met Glu Phe Lys Ala Met Asn Ala Lys Leu Leu Met Asp Pro Lys
        115                 120                 125
Arg Gln Ile Phe Leu Asp Gln Asn Met Leu Thr Ala Ile Asp Glu Leu
    130                 135                 140
Leu Gln Ala Leu Asn Phe Asn Ser Val Thr Val Pro Gln Lys Ser Ser
145                 150                 155                 160
Leu Glu Glu Pro Asp Phe Tyr Lys Thr Lys Ile Lys Leu Cys Ile Leu
                165                 170                 175
Leu His Ala Phe Arg Ile Arg Ala Val Thr Ile Asp Arg Met Met Ser
            180                 185                 190
Tyr Leu Asn Ser Ser
        195
<210> 2
<211> 307
<212> PRT
<213> 犬(Canis familiaris)
<400> 2
Ile Trp Glu Leu Glu Lys Asp Val Tyr Val Val Glu Leu Asp Trp His
1               5                   10                  15
Pro Asp Ala Pro Gly Glu Met Val Val Leu Thr Cys His Thr Pro Glu
            20                  25                  30
Glu Asp Asp Ile Thr Trp Thr Ser Ala Gln Ser Ser Glu Val Leu Gly
        35                  40                  45
Ser Gly Lys Thr Leu Thr Ile Gln Val Lys Glu Phe Gly Asp Ala Gly
    50                  55                  60
Gln Tyr Thr Cys His Lys Gly Gly Lys Val Leu Ser Arg Ser Leu Leu
65                  70                  75                  80
Leu Ile His Lys Lys Glu Asp Gly Ile Trp Ser Thr Asp Ile Leu Lys
                85                  90                  95
Glu Gln Lys Glu Ser Lys Asn Lys Ile Phe Leu Lys Cys Glu Ala Lys
            100                 105                 110
Asn Tyr Ser Gly Arg Phe Thr Cys Trp Trp Leu Thr Ala Ile Ser Thr
        115                 120                 125

Asp Leu Lys Phe Ser Val Lys Ser Ser Arg Gly Phe Ser Asp Pro Gln
    130                 135                 140
Gly Val Thr Cys Gly Ala Val Thr Leu Ser Ala Glu Arg Val Arg Val
145                 150                 155                 160
Asp Asn Arg Asp Tyr Lys Lys Tyr Thr Val Glu Cys Gln Glu Gly Ser
                165                 170                 175
Ala Cys Pro Ser Ala Glu Glu Ser Leu Pro Ile Glu Val Val Val Asp
            180                 185                 190
Ala Ile His Lys Leu Lys Tyr Glu Asn Tyr Thr Ser Ser Phe Phe Ile
        195                 200                 205
Arg Asp Ile Ile Lys Pro Asp Pro Pro Thr Asn Leu Gln Leu Lys Pro
    210                 215                 220
Leu Lys Asn Ser Arg His Val Glu Val Ser Trp Glu Tyr Pro Asp Thr
225                 230                 235                 240
Trp Ser Thr Pro His Ser Tyr Phe Ser Leu Thr Phe Cys Val Gln Ala
                245                 250                 255
Gln Gly Lys Asn Asn Arg Glu Lys Lys Asp Arg Leu Cys Val Asp Lys
            260                 265                 270
Thr Ser Ala Lys Val Val Cys His Lys Asp Ala Lys Ile Arg Val Gln
        275                 280                 285
Ala Arg Asp Arg Tyr Tyr Ser Ser Ser Trp Ser Asp Trp Ala Ser Val
    290                 295                 300
Ser Cys Ser
305
<210> 3
<211> 127
<212> PRT
<213> 犬(Canis familiaris)
<400> 3
Ala Pro Thr Arg Ser Pro Thr Leu Val Thr Arg Pro Ser Gln His Val
1               5                   10                  15
Asp Ala Ile Gln Glu Ala Leu Ser Leu Leu Asn Asn Ser Asn Asp Val
            20                  25                  30
Thr Ala Val Met Asn Lys Ala Val Lys Val Val Ser Glu Val Phe Asp
        35                  40                  45
Pro Glu Gly Pro Thr Cys Leu Glu Thr Arg Leu Gln Leu Tyr Lys Glu
    50                  55                  60
Gly Leu Gln Gly Ser Leu Thr Ser Leu Lys Asn Pro Leu Thr Met Met
65                  70                  75                  80
Ala Asn His Tyr Lys Gln His Cys Pro Pro Thr Pro Glu Ser Pro Cys
                85                  90                  95
Ala Thr Gln Asn Ile Asn Phe Lys Ser Phe Lys Glu Asn Leu Lys Asp
            100                 105                 110
Phe Leu Phe Asn Ile Pro Phe Asp Cys Trp Lys Pro Val Lys Lys
        115                 120                 125
<210> 4
<211> 135
<212> PRT
<213> 犬(Canis familiaris)
<400> 4
Ala Pro Ile Thr Ser Ser Ser Thr Lys Glu Thr Glu Gln Gln Met Glu
1               5                   10                  15
Gln Leu Leu Leu Asp Leu Gln Leu Leu Leu Asn Gly Val Asn Asn Tyr
            20                  25                  30
Glu Asn Pro Gln Leu Ser Arg Met Leu Thr Phe Lys Phe Tyr Thr Pro
        35                  40                  45
Lys Lys Ala Thr Glu Phe Thr His Leu Gln Cys Leu Ala Glu Glu Leu
    50                  55                  60

Lys Asn Leu Glu Glu Val Leu Gly Leu Pro Gln Ser Lys Asn Val His
65                  70                  75                  80
Leu Thr Asp Thr Lys Glu Leu Ile Ser Asn Met Asn Val Thr Leu Leu
                85                  90                  95
Lys Leu Lys Gly Ser Glu Thr Ser Tyr Asn Cys Glu Tyr Asp Asp Glu
            100                 105                 110
Thr Ala Thr Ile Thr Glu Phe Leu Asn Lys Trp Ile Thr Phe Cys Gln
        115                 120                 125
Ser Ile Phe Ser Thr Leu Thr
    130                 135
<210> 5
<211> 197
<212> PRT
<213> 猫(Felis catus)
<400> 5
Arg Asn Leu Pro Thr Pro Thr Pro Ser Pro Gly Met Phe Gln Cys Leu
1               5                   10                  15
Asn His Ser Gln Thr Leu Leu Arg Ala Ile Ser Asn Thr Leu Gln Lys
            20                  25                  30
Ala Arg Gln Thr Leu Glu Phe Tyr Pro Cys Thr Ser Glu Glu Ile Asp
        35                  40                  45
His Glu Asp Ile Thr Lys Asp Lys Thr Ser Thr Val Glu Ala Cys Leu
    50                  55                  60
Pro Leu Glu Leu Thr Met Asn Glu Ser Cys Leu Ala Ser Arg Glu Ile
65                  70                  75                  80
Ser Leu Ile Thr Asn Gly Ser Cys Leu Ala Ser Arg Lys Thr Ser Phe
                85                  90                  95
Met Thr Thr Leu Cys Leu Ser Ser Ile Tyr Glu Asp Leu Lys Met Tyr
            100                 105                 110
Gln Val Glu Phe Lys Ala Met Asn Ala Lys Leu Leu Met Asp Pro Lys
        115                 120                 125
Arg Gln Ile Phe Leu Asp Gln Asn Met Leu Thr Ala Ile Asp Glu Leu
    130                 135                 140
Leu Gln Ala Leu Asn Val Asn Ser Val Thr Val Pro Gln Asn Ser Ser
145                 150                 155                 160
Leu Glu Glu Pro Asp Phe Tyr Lys Thr Lys Ile Lys Leu Cys Ile Leu
                165                 170                 175
Leu His Ala Phe Arg Ile Arg Ala Val Thr Ile Asn Arg Met Met Ser
            180                 185                 190
Tyr Leu Asn Ser Ser
        195
<210> 6
<211> 307
<212> PRT
<213> 猫(Felis catus)
<400> 6
Ile Trp Glu Leu Glu Lys Asn Val Tyr Val Val Glu Leu Asp Trp His
1               5                   10                  15
Pro Asp Ala Pro Gly Glu Met Val Val Leu Thr Cys Asn Thr Pro Glu
            20                  25                  30
Glu Asp Asp Ile Thr Trp Thr Ser Asp Gln Ser Ser Glu Val Leu Gly
        35                  40                  45
Ser Gly Lys Thr Leu Thr Ile Gln Val Lys Glu Phe Ala Asp Ala Gly
    50                  55                  60
Gln Tyr Thr Cys His Lys Gly Gly Glu Val Leu Ser His Ser Phe Leu
65                  70                  75                  80
Leu Ile His Lys Lys Glu Asp Gly Ile Trp Ser Thr Asp Ile Leu Arg
                85                  90                  95

Glu Gln Lys Glu Ser Lys Asn Lys Ile Phe Leu Lys Cys Glu Ala Lys
            100                 105                 110
Asn Tyr Ser Gly Arg Phe Thr Cys Trp Trp Leu Thr Ala Ile Ser Thr
        115                 120                 125
Asp Leu Lys Phe Thr Val Lys Ser Ser Arg Gly Ser Ser Asp Pro Gln
    130                 135                 140
Glu Val Thr Cys Gly Ala Ala Thr Leu Ser Ala Glu Lys Val Arg Val
145                 150                 155                 160
Asp Asn Arg Asp Tyr Lys Lys Tyr Thr Val Glu Cys Gln Glu Gly Ser
                165                 170                 175
Ala Cys Pro Ala Ala Glu Glu Ser Leu Pro Ile Glu Val Val Val Asp
            180                 185                 190
Ala Ile His Lys Leu Lys Tyr Glu Asn Tyr Thr Ser Ser Phe Phe Ile
        195                 200                 205
Arg Asp Ile Ile Lys Pro Asp Pro Pro Lys Asn Leu Gln Leu Lys Pro
    210                 215                 220
Leu Lys Asn Ser Arg His Val Glu Val Ser Trp Glu Tyr Pro Asp Thr
225                 230                 235                 240
Trp Ser Thr Pro His Ser Tyr Phe Ser Leu Thr Phe Gly Val Gln Val
                245                 250                 255
Gln Gly Lys Asn Asn Arg Glu Lys Lys Asp Arg Leu Ser Val Asp Lys
            260                 265                 270
Thr Ser Ala Lys Val Val Cys His Lys Asp Ala Lys Ile Arg Val Gln
        275                 280                 285
Ala Arg Asp Arg Tyr Tyr Ser Ser Ser Trp Ser Asn Trp Ala Ser Val
    290                 295                 300
Ser Cys Ser
305
<210> 7
<211> 127
<212> PRT
<213> 猫(Felis catus)
<400> 7
Ala Pro Thr Ser Ser Pro Ser Ser Val Thr Arg Pro Trp Gln His Val
1               5                   10                  15
Asp Ala Met Lys Glu Ala Leu Ser Leu Leu Asn Asn Ser Ser Glu Ile
            20                  25                  30
Thr Ala Val Met Asn Glu Thr Val Glu Val Val Ser Glu Met Phe Asp
        35                  40                  45
Pro Glu Glu Pro Lys Cys Leu Gln Thr His Leu Lys Leu Tyr Glu Gln
    50                  55                  60
Gly Leu Arg Gly Ser Leu Ile Ser Leu Lys Glu Pro Leu Arg Met Met
65                  70                  75                  80
Ala Asn His Tyr Lys Gln His Cys Pro Leu Thr Pro Glu Thr Pro Cys
                85                  90                  95
Glu Thr Gln Thr Ile Thr Phe Lys Asn Phe Lys Glu Lys Leu Lys Asp
            100                 105                 110
Phe Leu Phe Asn Asn Pro Phe Asp Cys Trp Gly Pro Asp Gln Lys
        115                 120                 125
<210> 8
<211> 134
<212> PRT
<213> 猫(Felis catus)
<400> 8
Ala Pro Ala Ser Ser Ser Thr Lys Glu Thr Gln Gln Gln Leu Glu Gln
1               5                   10                  15
Leu Leu Leu Asp Leu Arg Leu Leu Leu Asn Gly Val Asn Asn Pro Glu
            20                  25                  30

Asn Pro Lys Leu Ser Arg Met Leu Thr Phe Lys Phe Tyr Val Pro Lys
        35                  40                  45
Lys Ala Thr Glu Leu Thr His Leu Gln Cys Leu Val Glu Glu Leu Lys
    50                  55                  60
Pro Leu Glu Glu Val Leu Tyr Leu Ala Gln Ser Lys Asn Phe His Leu
65                  70                  75                  80
Asn His Ile Lys Glu Leu Met Ser Asn Ile Asn Val Thr Val Leu Lys
                85                  90                  95
Leu Lys Gly Ser Glu Thr Arg Phe Thr Cys Asn Tyr Asp Asp Glu Thr
            100                 105                 110
Ala Thr Ile Val Glu Phe Leu Asn Lys Trp Ile Thr Phe Cys Gln Ser
        115                 120                 125
Ile Phe Ser Thr Leu Thr
    130

## Claims

1. An anti-tumor drug composition **characterized by** comprising proteins IL12, GMCSF, and IL2.

2. The anti-tumor drug composition according to claim 1, **characterized in that**, in the composition, a mass ratio of the IL12 protein: the GMCSF protein: the IL2 protein is in ranges of 0.1-10:0.1-10:0.1-10.

3. The anti-tumor drug composition according to claim 2, **characterized in that**, in the composition, the mass ratio of the IL12 protein: the GMCSF protein: the IL2 protein is in ranges of 0.6-6:0.6-6:0.6-6.

4. The anti-tumor drug composition according to claim 3, **characterized in that**, in the composition, the mass ratio of the IL12 protein: the GMCSF protein: the IL2 protein is 1:6:2.

5. A polynucleotide composition, **characterized in that**, the polynucleotide composition comprises one of:
(a) a polynucleotide composition coding proteins IL12, GMCSF, and IL2 in claim 1; and
(b) a polynucleotide composition complementary to the polynucleotide (a).

6. A recombinant vector composition, **characterized by** being constructed from each polynucleotide of the polynucleotide composition in claim 5 with a plasmid, a virus, and an expressing vector, respectively.

7. A genetically engineered host cell composition, **characterized in that**, a the host cell of the host cell composition comprises one of:
(a) a host cell transformed or transduced from the recombinant vector in claim 6; and
(b) a host cell transformed or transduced from the polynucleotide composition in claim 5.

8. An anti-tumor drug, **characterized by** comprising the anti-tumor drug composition in any of claims 1-4, or the polynucleotide composition in claim 5, or the recombinant vector composition in claim 6, or the host cell composition in claim 7, and a pharmaceutically acceptable carrier or buffer or additive or excipient or adjuvant.

9. A use of the anti-tumor drug composition in any of claims 1-4, or the polynucleotide composition in claim 5, or the recombinant vector composition in claim 6, or the host cell composition in claim 7, or the anti-tumor drug in claim 8 in a field of a drug for treating a mammalian tumor.

10. The use of the anti-tumor drug composition in any of claims 1-4, or the polynucleotide composition in claim 5, or the recombinant vector composition in claim 6, or the host cell composition in claim 7, or the anti-tumor drug in claim 8 in the field of the drug for treating the mammalian tumor according to claim 9, **characterized in that**, the mammalia comprises Diprotodontia other than human beings, Carnivora, Perissodactyla, Artiodactyla, Rodentia, and Lagomorpha.

11. The use of the anti-tumor drug composition in any of claims 1-4, or the polynucleotide composition in claim 5, or the recombinant vector composition in claim 6, or the host cell composition in claim 7, or the anti-tumor drug in claim 8 in the field of the drug for treating the mammalian tumor according to claim 10, **characterized in that** the tumor comprises melanoma, kidney cancer, prostate cancer, breast cancer, colon cancer, lung cancer, liver cancer, bone cancer, pancreatic cancer, skin cancer, head or neck cancer, oral cancer, nasopharyngeal cancer, skin or intraocular malignant melanoma, uterine cancer, ovarian cancer, rectal cancer, anal cancer, gastric cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, thyroid cancer, parathyroid cancer, adrenal cancer, soft tissue sarcoma, urethral cancer, penile cancer, acute myeloid leukemia, chronic myeloid leukemia, acute lymphoblastic leukemia, chronic lymphocytes leukemia, lymphocytic lymphoma, bladder cancer, renal or ureteral cancer, renal pelvis cancer, central nervous system neoplasm, primary central nervous system lymphoma, tumor angiogenesis, spinal cord tumor, brain stem glioma, pituitary adenoma, Kaposi's sarcoma, epidermoid carcinoma, squamous cell carcinoma, and T cell lymphoma.

12. The anti-tumor drug composition in any of claims 1-4, or the polynucleotide composition in claim 5, or the recombinant vector composition in claim 6, or the host cell composition in claim 7, or the anti-tumor drug in claim 8, **characterized by** being injected into an affected area of animal or introduced into an affected area of a tumor by intravenous injection through a targeted carrier.
